# FASCICULE DE BREVET EUROPEEN

(11) **EP 2 252 221 B1**
(45) Date de publication et mention de la délivrance du brevet: **21.09.2011**
(21) Numéro de dépôt: 09710535.7
(22) Date de dépôt: 12.02.2009
(51) Int. Cl.: A61B 17/28, A61B 18/14

(54) **DISPOSITIF DE CLAMP POUR PINCE CHIRURGICALE**
KLEMMVORRICHTUNG FÜR CHIRURGISCHE ZANGEN
CLAMP DEVICE FOR SURGICAL PLIERS

(30) Priorité: 13.02.2008 FR 0800774
(43) Date de publication de la demande: 24.11.2010
(73) Titulaire: Noury, Guillaume, 78670 Villennes sur Seine (FR)
(72) Inventeur: Noury, Guillaume, 78670 Villennes sur Seine (FR)
(74) Mandataire: CAPRI
(86) Numéro de dépôt international: PCT/FR2009/050224
(87) Numéro de publication internationale: WO 2009/101364

(56) Documents cités:
- DE-C- 273 689
- DE-U1-202005 020 964
- US-A1- 2007 167 977
- US-B1- 6 187 003

## Description

La présente invention concerne un dispositif de clamp pour pince chirurgicale, et en particulier pour pince électro-chirurgicale.

Les pinces chirurgicales sont, comme leur nom l'indique, utilisées en chirurgie. Elles comportent généralement deux branches, similaires à des ciseaux, que le chirurgien peut amener en position de fermeture, afin de refermer les mors de la pince autour d'une cible. Lorsque la pince est une pince électro-chirurgicale, ladite pince est alimentée par un courant électrique qui permet en position de fermeture des mors, de chauffer et de transformer les tissus maintenus entre les mors fermés. Lorsque les deux mors de la pince ont des polarités différentes, la pince est dite bipolaire. Les pinces chirurgicales incorporent souvent un dispositif de clamp, c'est-à-dire un dispositif de blocage qui permet de maintenir la pince en position fermée, sans que le chirurgien soit obligé de garder ladite pince en main. Généralement, ces dispositifs comprennent un système de cran prévu entre les deux branches de la pince. Cette mise en oeuvre engendre un certain nombre d'inconvénients. Ainsi, à chaque fois que le chirurgien ferme la pince, le dispositif de clamp s'enclenche, de sorte qu'il n'y a aucune possibilité pour le chirurgien d'utiliser la pince sans le dispositif de clamp. De plus, le clamp doit avoir une résistance mécanique élevée pour résister aux contraintes générées lorsque les mors sont fermés. Ces dispositifs de clamp sont ainsi intégrés de manière monobloc à la pince, et sont donc réalisés dans la même matière métallique que le reste de la pince. Or, la réalisation d'un clamp en métal, en particulier en acier, interconnecte les deux branches lorsque le clamp est enclenché, ce qui revient à mettre les deux mors en court-circuit. Ceci rend impossible l'utilisation d'une pince bipolaire, dont les mors sont par définition à des polarités différentes. Pour alimenter chaque mors séparément, les pinces électro-chirurgicales qui existent aujourd'hui avec un dispositif de clamp ont ainsi une polarité sur l'une des deux branches et le deuxième mors est totalement isolé de la branche sur laquelle il est fixé, ce mors étant alimenté directement par un fil encastré dans ladite branche et qui est relié à un connecteur prévu sur les anneaux de ladite pince. Cette mise en oeuvre, qui consiste à isoler l'un des deux mors de la branche sur laquelle il est fixé, est complexe et donc coûteuse. Les documents US 2007/167977, DE 20 2005 020964 U1 et DE 273 689 C décrivent des pinces non électriques et le document US-6 187 003 décrit une pince électrique pourvu d'un clamp non amovible.

La présente invention a pour but de fournir un dispositif de clamp pour pince chirurgicale qui ne reproduit pas les inconvénients susmentionnés.

En particulier, la présente invention a pour but de fournir un dispositif de clamp pour pince chirurgicale qui permet d'utiliser ladite pince avec ou sans ledit dispositif de clamp selon les besoins du chirurgien.

La présente invention a aussi pour but de fournir un dispositif de clamp pour pince chirurgicale permettant l'utilisation d'une pince bipolaire dans laquelle les deux branches sont alimentées séparément.

La présente invention a encore pour but de fournir un dispositif de clamp pour pince chirurgicale qui soit simple et peu coûteux à fabriquer et à assembler, modulable dans son utilisation et son fonctionnement, et qui s'adapte à différents types de pinces, notamment une pince électro-chirurgicale.

La présente invention a donc pour objet un dispositif de clamp pour pince chirurgicale, comprenant un premier élément de clamp pourvu de premiers moyens de blocage, un second élément de clamp pourvu de seconds moyens de blocage, lesdits premier et second moyens de blocage étant adaptés à coopérer, en position de blocage, pour bloquer la pince en position fermée, caractérisé en ce que chacun desdits premier et second éléments de clamp comporte des moyens de fixation pour une fixation amovible sur une branche respective de la pince, et en ce que chaque élément de clamp comporte une connexion électrique pour alimenter en courant électrique la branche respective de la pince sur laquelle il est monté.

Des modes de réalisation avantageux sont décrits dans les revendications dépendantes.

La présente invention a aussi pour objet un ensemble constitué d'une pince chirurgicale comportant deux branches, et d'un dispositif de clamp tel que décrit ci-dessus.

Avantageusement, ladite pince est bipolaire.

Ces caractéristiques et avantages et d'autres de la présente invention apparaîtront plus clairement au cours de la description détaillée suivante d'un mode de réalisation avantageux de celle-ci, faite en référence aux dessins joints, donnés à titre d'exemples non limitatifs, et sur lesquels :
- la figure 1 est une vue schématique en perspective d'un dispositif de clamp fixé sur une pince électro-chirurgicale, en position de blocage,
- la figure 2 est une vue similaire à celle de la figure 1, avec le dispositif de clamp dans une position de non utilisation, même en position fermée de la pince,
- la figure 3 est une vue schématique en perspective d'un des deux éléments de clamp du dispositif de clamp représentés sur les figures 1 et 2, et
- la figure 4 est une vue schématique en perspective d'une pince chirurgicale sur laquelle le dispositif de clamp selon l'invention peut être fixé de manière amovible.

En référence aux figures, il est représenté un exemple de pince chirurgicale utilisable avec la présente invention. Il est entendu que toutes pinces chirurgicales, même de formes différentes, pourraient être utilisées.

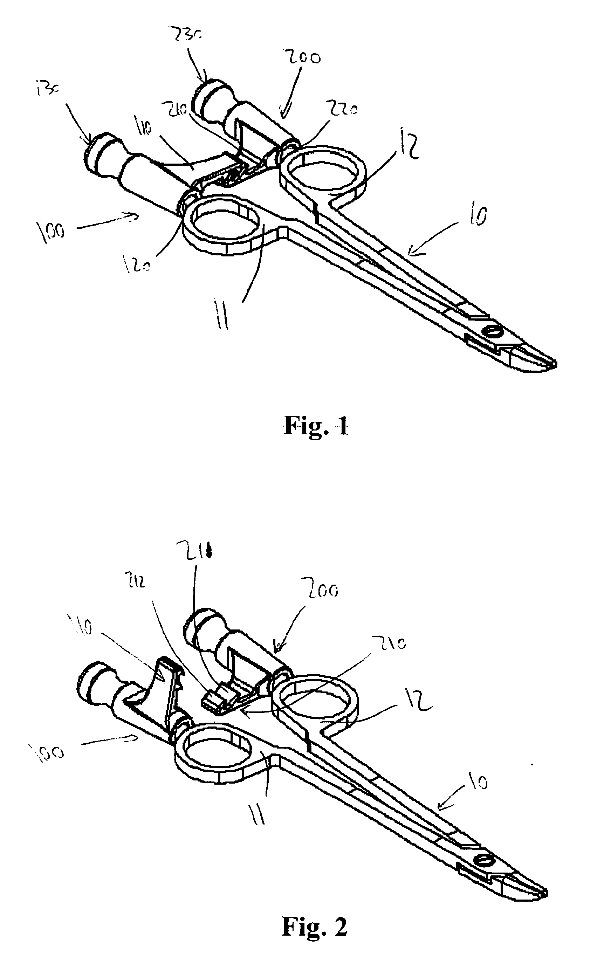

Le dispositif de clamp comporte un premier élément de clamp 100 et un second élément de clamp 200. De préférence, ces premier et second éléments de clamp sont identiques, ce qui simplifie la fabrication et l'utilisation du dispositif. Chaque élément de clamp comporte des moyens de blocage, des premiers moyens de blocage 110 pour le premier élément de clamp 100, et des seconds moyens de blocage 210 pour le second élément de blocage 200. Lorsque le dispositif de clamp est assemblé sur une pince chirurgicale, ces moyens de blocage coopèrent les uns avec les autres pour maintenir la pince en position fermée dans la position de blocage, comme représenté sur la figure 1. Selon l'invention, les éléments de clamp 100, 200 sont adaptés à être fixés de manière amovible à la pince 10. Plus particulièrement, le premier élément de clamp 100 est fixé de manière amovible à une première branche 11 de la pince 10 et le second élément de clamp 200 est fixé de manière amovible à une seconde branche 12 de la pince 10.

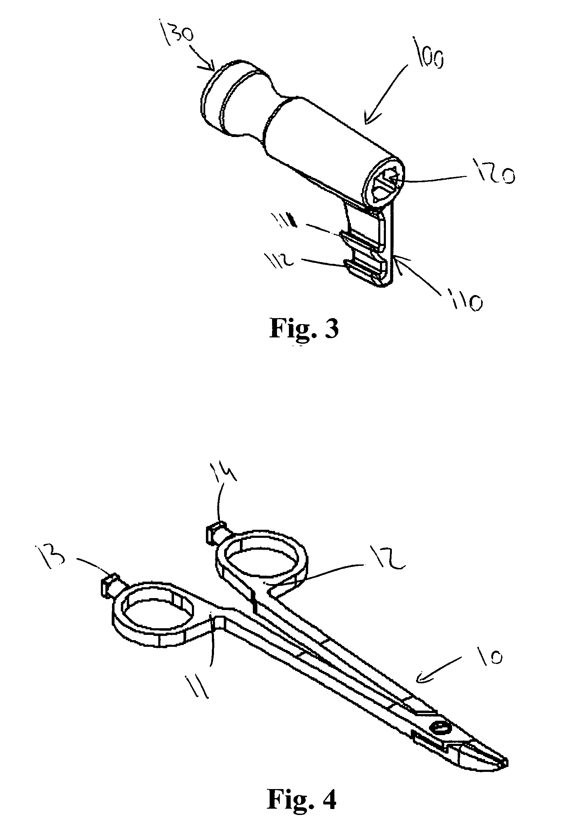

Les moyens de fixation amovibles sont plus clairement visibles sur les figures 3 et 4. Ainsi, chacun des éléments de clamp 100, 200 comporte des moyens de fixation respectifs 120, 220 permettant d'être fixé de manière amovible à une branche respective 11, 12 de la pince 10. Ces moyens de fixation peuvent notamment comporter une prise femelle réalisée dans l'élément de clamp 100, 200, cette prise femelle étant adaptée à coopérer avec une prise mâle de forme correspondante 13, 14 qui peut être prévue sur les branches 11, 12 de la pince 10. Plus particulièrement, comme visible sur les figures 3 et 4, la prise femelle 120 comporte un orifice de forme polygonale, de préférence de forme environ carrée, et la prise mâle 13, 14 sur la branche 11, 12 respective de la pince 10 comporte une projection de forme polygonale complémentaire à l'orifice polygonale 120 de l'élément de clamp respectif, et donc dans l'exemple représenté, une projection de forme environ carrée. Avantageusement, la fixation de l'élément de clamp sur la branche respective de la pince se fait par encliquetage de la projection 13, 14 dans l'orifice carré 120, 220 de l'élément de clamp respectif. Cette mise en oeuvre est particulièrement avantageuse puisque selon la position d'assemblage de l'élément de clamp sur sa branche respective, le dispositif de clamp est actif ou non actif. Ainsi, comme représenté sur la figure 1, les deux éléments de clamp coopèrent en position de blocage pour bloquer la pince 10 en position fermée. Au contraire, sur la figure 2, l'élément de clamp 100 est tourné de 90° autour de la projection carrée 13 de la branche 11, de sorte qu'en position fermée de la pince 10, le dispositif de clamp ne s'enclenche pas et il n'y a pas de blocage de la pince en position fermée. Le chirurgien peut donc de manière très simple, par simple rotation d'un des deux éléments de clamp, activer ou désactiver le dispositif de clamp. Comme visible sur les figures, les moyens de fixation 110, 210 de chaque élément de clamp 100, 200 comportent au moins une, de préférence deux projections 111, 112 (respectivement 211, 212), pour assurer un blocage sûr comme représenté sur la figure 1. Bien entendu d'autres moyens de blocage pourraient être envisagés pour réaliser le blocage en position de fermeture de la pince 10.

Lorsque la pince 10 est une pince électro-chirurgicale, elle est alimentée en courant électrique. A cet égard, chaque élément de clamp comporte une connexion électrique 130, 230 permettant le branchement d'une alimentation électrique. L'avantage de l'invention est de permettre l'utilisation d'une pince bi-polaire, chaque élément de clamp 100, 200 permettant de brancher une alimentation respective pour la branche 11, 12 respective de la pince 10. Chaque élément de clamp 100, 200 comporte à cet effet une partie interne en matériau conducteur, notamment en métal, et une partie externe en matériau isolant, notamment en matière plastique, les moyens de fixation étant réalisés sur la partie externe. Ainsi, en position de blocage, lorsque les deux éléments de clamp coopèrent, ils sont électriquement isolés l'un de l'autre, empêchant tout court-circuit. La présente invention permet donc d'utiliser une pince bipolaire, où chaque mors est directement alimenté avec une polarité différente, avec les avantages qui en découlent. De préférence, la partie externe en plastique est surmoulée sur la partie interne en métal, offrant ainsi une sécurité maximale à l'utilisateur. Ainsi, avec une pince électro-chirurgicale, chaque élément de clamp 100, 200 est fixé de manière amovible sur une branche respective 11, 12 de la pince électro-chirurgicale 10, ladite fixation étant électriquement conductrice. Au niveau de la connexion électrique 130, 230, le câble d'alimentation (non représenté) peut être branché d'une manière classique.

Un autre avantage de la présente invention est de pouvoir réaliser un dispositif de clamp qui puisse être adapté à diverses pinces chirurgicales. Il suffit à cet égard de prévoir une gamme de pinces comportant toutes des projections 13, 14 adaptées aux moyens de fixation 120, 220 des éléments de clamp 100, 200 pour pouvoir appliquer le même dispositif de clamp sur différentes pinces chirurgicales. La présente invention permet donc de réaliser des dispositifs réutilisables et modulables, ce qui est avantageux en matière de coût de fabrication et d'utilisation.

En variante au mode de réalisation représenté, où les éléments de clamp sont fixés sur des projection prévues aux extrémités arrières de chaque branche, ces éléments de clamp pourraient aussi être fixés de manière amovible aux branches de la pince à des endroits différents. Par exemple, des éléments de clamp en matière plastique pourraient être fixés sur des projections prévues sur les cotés internes qui se font face des deux branches de la pince.

D'autres modifications peuvent être apportées par l'homme de l'art, sans sortir du cadre de la présente invention tel que défini par les revendications annexées.

## Revendications

1. Dispositif de clamp pour pince chirurgicale (10), comprenant un premier élément de clamp (100) pourvu de premiers moyens de blocage (110), un second élément de clamp (200) pourvu de seconds moyens de blocage (210), lesdits premier et second moyens de blocage (110, 210) étant adaptés à coopérer, en position de blocage, pour bloquer la pince (10) en position fermée, chacun desdits premier et second éléments de clamp (100, 200) comportant des moyens de fixation (120, 220) pour une fixation amovible sur une branche respective (11, 12) de la pince (10), **caractérisé en ce que** chaque élément de clamp (100, 200) comporte une connexion électrique (130, 230) pour alimenter en courant électrique la branche respective (11, 12) de la pince (10) sur laquelle il est monté.

2. Dispositif selon la revendication 1, dans lequel lesdits moyens de fixation comportent une prise femelle (120, 220) coopérant avec une prise mâle correspondante (13, 14) prévue sur la branche respective (11, 12) de la pince (10).

3. Dispositif selon la revendication 2, dans lequel ladite prise femelle (120, 220) comporte un orifice de forme polygonale, de préférence environ carrée, ladite prise mâle (13, 14) comportant une projection de forme complémentaire, adaptée à se fixer de manière amovible dans ladite prise femelle.

4. Dispositif selon l'une quelconque des revendications précédentes, dans lequel ladite fixation amovible des éléments de clamp (100, 200) sur les branches (11, 12) de la pince (10) est réalisée par encliquetage.

5. Dispositif selon l'une quelconque des revendications précédentes, dans lequel lesdits premier et second moyens de blocage (110, 210) comportent respectivement au moins une projection saillante (111, 112 ; 211, 212), lesdites projections saillantes coopérant les unes avec les autres en position de blocage.

6. Dispositif selon l'une quelconque des revendications précédentes, dans lequel la fixation amovible des premier et second éléments de clamp (100, 200) sur une branche respective (11, 12) de la pince (10) est électriquement conductrice.

7. Dispositif selon l'une quelconque des revendications précédentes, dans lequel chaque élément de clamp (100, 200) comporte une partie interne électriquement conductrice, notamment métallique, et une partie externe non conductrice, notamment en matière plastique, la partie externe incorporant les moyens de blocage (110, 210), de sorte que en position de blocage, lesdits premier et second éléments de clamp (100, 200) sont électriquement isolés l'un de l'autre.

8. Dispositif selon la revendication 7, dans lequel ladite partie externe est surmoulée sur la partie interne.

9. Dispositif selon l'une quelconque des revendications précédentes, dans lequel lesdits premier et second éléments de clamp (100, 200) sont identiques.

10. Ensemble constitué d'une pince chirurgicale (10) comportant deux branches (11, 12), et d'un dispositif de clamp selon l'une des revendications précédentes.

11. Ensemble selon la revendication 10, dans lequel ladite pince (10) est bipolaire.

## Claims

1. A clamp device for surgical pliers (10), comprising a first clamp member (100) provided with first blocking means (110), a second clamp member (200) provided with second blocking means (210), said first and second blocking means (110, 210) being adapted to cooperate, in a blocking position, to block the pliers (10) in a closed position, each of said first and second clamp members (100, 200) comprising fixing means (120, 220) for removable attachment on a respective arm (11, 12) of the pliers (10), **characterised in that** each clamp member (100, 200) comprises an electrical connection (130, 230) for supplying electric current to the respective arm (11, 12) of the pliers (10) on which they are mounted.

2. The device as claimed in Claim 1, in which said fixing means comprise a female socket (120, 220) cooperating with a corresponding male socket (13, 14) provided on the respective arm (11, 12) of the pliers (10).

3. The device as claimed in Claim 2, in which said female socket (120, 220) comprises an orifice in polygonal form, preferably square, said male socket (13, 14) comprising a projection of complementary form adapted to be attached removably in said female socket.

4. The device as claimed in any of the preceding claims, in which said removable attachment of the clamp members (100, 200) on the arms (11, 12) of the pliers (10) is done via snap-locking.

5. The device as claimed in any of the preceding claims, in which said first and second blocking means (110, 210) respectively comprise at least one salient projection (111, 112; 211, 212), said salient projections cooperating with one another in a blocking position.

6. The device as claimed in any of the preceding claims, in which the removable attachment of the first and second clamp members (100, 200) on a respective arm (11, 12) of the pliers (10) is electrically conductive.

7. The device as claimed in any of the preceding claims, in which each clamp member (100, 200) comprises an electrically conductive internal part, especially metallic, and a nonconductive external part, especially made of plastic, the external part incorporating the blocking means (110, 210), such that when in the blocking position said first and second clamp members (100, 200) are electrically insulated from one another.

8. The device as claimed in Claim 7, in which said external part is moulded on the part internal.

9. The device as claimed in any of the preceding claims, in which said first and second clamp members (100, 200) are identical.

10. An assembly comprising surgical pliers (10) comprising two arms (11, 12), and a clamp device as claimed in any of the preceding claims.

11. The assembly as claimed in Claim 10, in which said pliers (10) are bipolar.

## Patentansprüche

1. Klemmvorrichtung für chirurgische Zangen (10), aufweisend ein erstes Klemmelement (100), das mit ersten Sperrmitteln (110) versehen ist, ein zweites Klemmelement (200), das mit zweiten Sperrmitteln (210) versehen ist, wobei die ersten und die zweiten Sperrmittel (110, 210) dazu geeignet sind, in der Sperrposition zusammenzuwirken, um die Zange (10) in der geschlossenen Position zu sperren, wobei jedes des ersten und zweiten Klemmelementes (100, 200) Befestigungsmittel (120, 220) zur lösbaren Befestigung auf einem jeweiligen Griff (11, 12) der Zange (10) aufweist, **dadurch gekennzeichnet, dass** jedes Klemmelement (100, 200) einen Stromanschluss (130, 230) aufweist, um den jeweiligen Griff (11, 12) der Zange (10), auf dem er montiert ist, mit Strom zu versorgen.

2. Vorrichtung nach Anspruch 1, wobei die Befestigungsmittel eine Steckdose (120, 220) aufweisen, die mit einem Stecker (13, 14) zusammenwirkt, der auf dem entsprechenden Griff (11, 12) der Zange (10) vorgesehen ist.

3. Vorrichtung nach Anspruch 2, wobei die Steckdose (120, 220) eine polygonale, vorzugsweise in etwa viereckige Öffnung aufweist, wobei der Stecker (13, 14) einen Vorsprung von komplementärer Form aufweist, der dazu geeignet ist, lösbar in der Steckdose befestigt zu werden.

4. Vorrichtung nach einem der vorhergehenden Ansprüche, wobei die lösbare Befestigung der Klemmelemente (100, 200) auf Griffen (11, 12) der Zange (10) durch Einrasten realisiert ist.

5. Vorrichtung nach einem der vorhergehenden Ansprüche, wobei die ersten und zweiten Sperrmittel (110, 210) jeweils mindestens einen Vorsprung (111, 112; 211, 212) aufweisen, wobei die Vorsprünge in der Sperrposition zusammenwirken.

6. Vorrichtung nach einem der vorhergehenden Ansprüche, wobei die lösbare Befestigung des ersten und des zweiten Klemmelementes (100, 200) auf einem jeweiligen Griff (11, 12) der Zange (10) elektrisch leitend ist.

7. Vorrichtung nach einem der vorhergehenden Ansprüche, wobei jedes Klemmelement (100, 200) einen elektrisch leitenden, insbesondere metallischen inneren Teil und einen nicht leitenden äußeren Teil, insbesondere aus Kunststoffmaterial, aufweist, wobei der äußere Teil die Sperrmittel (110, 210) umfasst, so dass in der Sperrposition das erste und das zweite Klemmelement (100, 200) voneinander elektrisch isoliert sind.

8. Vorrichtung nach Anspruch 7, wobei der äußere Teil auf dem inneren Teil ausgegossen ist.

9. Vorrichtung nach einem der vorhergehenden Ansprüche, wobei das erste und das zweite Klemmelement (100, 200) identisch sind.

10. Satz, bestehend aus einer chirurgischen Zange (10), die zwei Griffe (11, 12) aufweist, und aus einer Klemmvorrichtung nach einem der vorhergehenden Ansprüche.

11. Satz nach Anspruch 10, wobei die Zange (10) zweipolig ist.
